# EUROPÄISCHE PATENTSCHRIFT

(11) **EP 0 947 216 B1**
(45) Veröffentlichungstag und Bekanntmachung des Hinweises auf die Patenterteilung: **22.12.2004**
(21) Anmeldenummer: 99250100.7
(22) Anmeldetag: 31.03.1999
(51) Int. Cl.: A61N 1/365

(54) **Vorrichtung zur Herzstimulation**
Cardiac stimulating device
Appareil de stimulation cardiaque

(30) Priorität: 31.03.1998 DE 19815540
(43) Veröffentlichungstag der Anmeldung: 06.10.1999
(73) Patentinhaber: BIOTRONIK Mess- und Therapiegeräte GmbH & Co Ingenieurbüro Berlin, 12359 Berlin (DE)
(72) Erfinder: Hastings, David, Lake Oswego, OR 97034 (US); Schaldach, Max, Prof.-Dr., 91054 Erlangen (DE); Rolison, Gary, Portland, OR 97229 (US); Weyant, Robert, R., Dureham, OR 97224 (US); Hutten, Prof.Dr.Helmut, A-8010 Graz (AT)
(74) Vertreter: Eisenführ, Speiser & Partner

(56) Entgegenhaltungen:
- EP-A- 0 770 407
- EP-A- 0 793 978
- US-A- 4 899 752
- US-A- 5 143 065
- US-A- 5 355 894
- US-A- 5 645 575

## Beschreibung

Die Erfindung betrifft eine Vorrichtung gemäß dem Oberbegriff des Anspruchs 1. Aus dem Stand der Technik sind vielfältige Vorrichtungen bekannt, welche mindestens einen innerhalb des Körpers des Patienten angeordneten Sensor zur Aufnahme eines eine Information über den Herzleistungsbedarf enthaltenden körpereigenen Meßsignals aufweisen, um daraus unter anderem ein Signal für die Beeinflussung der Stimulationsrate herzuleiten.

Die bisherigen Vorrichtungen gingen davon aus, daß eine unmittelbare und zeitgerechte Beziehung zwischen der Veränderung des eine Information über den Herzleistungsbedarf beinhaltenden körpereigenen Meßsignals und der zu beeinflussenden Stimulationsgröße besteht, wie beispielsweise der Amplitude, der Frequenz oder mittleren Ereignishäufigkeit von durch einen Sensor aufgenommenen Signalen und der Herzrate.

Es hat sich jedoch gezeigt, daß eine derartige unmittelbare Verarbeitung im Zeitoder Frequenzbereich häufig nicht zu den gewünschten Ergebnissen führt.

Aus der US 5,143,065 ist ein Herzschrittmacher bekannt, bei der die Ratensteuerung innerhalb von Schlafphasen und Wachphasen des zirkadianischen Zyklusses unterschiedlich erfolgt.

Der Erfindung liegt deshalb die Aufgabe zugrunde, die Steuerung von Stimulationsereignissen und insbesondere der Herzrate zu verbessern. Die Aufgabe wird, ausgehend von einer Vorrichtung gemäß dem Oberbegriff des Anspruchs 1, durch die im kennzeichnenden Teil des Anspruchs 1 angegebenen Merkmale gelöst. Eine Vorrichtung gemäß Oberbegriff ist aus US 5,645,575 bekannt.

Die Erfindung schließt die technische Lehre ein, daß bei der Ermittlung einer die Folge des Stimulationssignals beeinflussenden Größe auch solche Zusammenhänge beachtet werden müssen, welche über eine unmittelbare Zuordnung hinausgehen.

Dazu gehört einerseits eine Transformation der zu berücksichtigenden Zeiträume in der Weise, daß die zeitliche Beobachtung auch über zurückliegende Zeiträume erfolgt und die Steuerung der Herzrate durch das wiederholte Auftreten von komplexen Frequenz- oder Ereignisfolgen kontrolliert wird, welche eine eigene Periodizität oder Rhythmik aufweisen. Die Ereignisse selbst werden durch Mustervergleich identifiziert. Die Periodizität der Frequenz- oder Ereignisfolgen weichen dabei deutlich von der natürlichen Herzrate ab und liegt im Bereich von mehreren Sekunden bis zu Tagen oder Monaten.

Auf diese Weise gelingt es, eine körpereigene Rhythmik zu "entschlüsseln" und der Herzstimulation zugänglich zu machen, welche bisher zwar schon bei der Steuerung der Vasomotorik des menschlichen Körpers eine wichtige Rolle spielte, aber für die Wiederherstellung des natürlichen Herzrhythmus nicht eingesetzt werden konnte.

Bei den auszuwertenden Signalen handelt es sich um Kreislaufgrößen, deren ständiger Wechsel in rhythmischen Schwankungen auch eine Information für den Herzleistungsbedarf enthält. Hierbei handelt es sich insbesondere um rhythmische Schwankungen des intraarteriellen Drucks oder aber um an verschiedenen Meßorten im Gefäßsystem aufgenommene Druckdifferenzen, welche ein Maß für den Gefäßwiderstand zwischen den Meßorten bilden.

Bei dem dem Blutdruck überlagerten rhythmischen Schema handelt es sich um Wellenzüge unterschiedlicher Ordnung, welche durch ihre Periodizität - und damit über die zu ihrer Detektion eingesetzten digitalen Filter- oder Korrelationsverfahren oder entsprechend angepaßte periodischen Meßzeitsegmente, welche den jeweiligen Amplitudenverlauf und/oder das zugeordnete Frequenzmuster erfassen, ermittelt werden können.

Mit der Erfindung ist der wichtige Vorteil verbunden, daß erstmals Signale, welche bisher nicht detektierbar waren oder unbeachtet blieben, die Stimulationsrate mitbestimmen, so daß auf die körperliche Aktivität des Patienten aufnehmende Sensormittel verzichtet werden kann.

Die Anwendung der erfindungsgemäßen Vorrichtung zur Aufrechterhaltung oder Wiederherstellung eines natürlichen Herzrhythmus durch Erzeugung eines elektrischen Stimulationssignals bezieht sich dabei insbesondere auf implantierbare Herzschrittmacher zur Behandlung von Brady- oder Tachykardien sowie entsprechende Rhythmuskorrekturgeräte, welche auch als implantierbare Defibrillatoren im Gebrauch sind.

Wenn gemäß der Erfindung einem Sensor eine Verarbeitungseinheit nachgeschaltet ist, welche aus dem Meßsignal ein körpereigenes Rhythmussignal gewinnt, dessen Periodizität mindestens gleich der Periodität der Atemtätigkeit ist und somit oberhalb der Periodizität der Herztätigkeit gelegen ist, wobei das körpereigene Rhythmussignal mindestens mittelbar ein Steuersignal bildet, das den Zeitpunkt oder die Zeitfolge und/oder den Zeitpunkt des Stimulationssignals beeinflußt, so bedeutet dies, daß aus einem dem Kreislauf- oder Nervensystem eingeprägten Rhythmussignal eine Information entnommen wird, welche für die Herztätigkeit von Bedeutung ist. Es wurde gefunden, daß diese Signale auch für die natürliche Herztätigkeit des Menschen von Bedeutung sind und eine Stimulation in Korrelation zu derartigen Signale mindestens eine physiologische Ergänzung zu auf andere Weise aus dem Körper des Patienten abgeleiteten, für die Herzfunktion relevanten Signalen bildet.

Eine wesentliche Anwendung besteht dabei insbesondere in einer der " inneren Uhr" des Patienten angepaßten Stimulationsrate entsprechend einem physiologischen Tag-Nacht-Rhythmus. Vielfach erweist es sich aber auch als günstig, wenn Kreislaufrhythmen oder anderen biologischen Rhythmen des Patienten in der Stimulation gefolgt wird. Eine derartige Stimulation entsprechend einem biologischen Rhythmus darf nicht verwechselt werden mit solchen "biologischen Rhythmen", welche tabellarisch ausgedruckt sind oder aber von Computern ohne näheren Bezug zu dem realen biologischen Objekt rein willkürlich nach Art eines Horoskops vorgegeben werden.

Schnellere Körperrhythmen erfolgten in einer Periodtät, die mit der der Atemtätigkeit beginnt und einem Rhythmus von im wesentlichen einem 6 bis 20 Sek, insbesondere 10 Sek-Rhythmus entspricht, umfaßt. Diese Rhythmen sind aber wohlgemerkt langsamer als die tatsächliche Herztätigkeit und stehen vorzugsweise in einem Frequenzuntersetzungsverhältnis dazu.

Die Selektion des körpereigenen Rhythmussignals kann zweckmäßigerweise mittels Digitalfilterung oder der Anwendung einer Korrelationstechnik erfolgen, wobei auch die Synchronisation entsprechender Phasenregelkreise (PLL) eine hohe Empfindlichkeit erlaubt.

Bei einer digitalen Verarbeitung besteht in vorteilhafter Weise auch die Möglichkeit, eine Detektion bzw. Synchronisation periodischer oder nichtperiodischer Vorgänge durch den Vergleich vollständiger Signalmuster im Amplituden- und/oder Frequenzbereich zu bewirken. Hierbei wird insbesondere in der Verarbeitungseinheit innerhalb eines mindestens mehrere Sekunden umfassenden Zeitsegments vorgegebener Dauer mindestens ein periodisch auftretendes Signal oder ein entsprechender Signalanteil mit einem charakteristischen Amplitudenverlaufs- oder Frequenzmuster mittels eines Zeitfensters selektiert und aus der Folge des Auftretens dieses Musters das körpereigene Rhythmussignal abgeleitet, welches die Zeitfolge oder den Zeitpunkt des Stimulationssignals beeinflußt. Die Breite der bei dieser Verarbeitung zu benutzenden Zeitsegmente entspricht dabei höchstens den Periodendauern der erwarteten Signale oder Signalanteile. Eine auf der Ähnlichkeit oder Übereinstimmung von in den Zeitsegmenten erscheinenden Amplituden- oder Frequenzmustern basierende Ermittlung eines Steuersignals beinhaltet den Vorteil, daß eine Ähnlichkeit der die Rhythmusfrequenz bestimmenden Signalanteile auch bei weniger sich wiederholenden Perioden schneller und sicherer zu erkennen ist und nicht das "Einschwingen" von Filterschaltungen oder dergleichen abgewartet werden muß, um die periodischen Signalanteile zu detektieren. Dies ist inbesondere günstig bei Rhythmen mit langer Wiederholzeit.

Zur Detektion von übereinstimmenden Signalmustern wird dabei insbesondere das jeweils in dem Zeitsegment aufgetretene periodische Signal oder ein entsprechender Signalanteil miteinem charakteristischen Amplitudenverlaufs- oder Frequenzmuster gespeichert, wobei das Muster des laufenden Zeitsegments mit mindestens einem gleichartigen, zuvor aufgenommenen, periodisch auftretenden Signal oder einem entsprechenden Signalanteil miteinem charakteristischen Amplitudenverlaufs- oder Frequenzmuster fortlaufend miteinander verglichen wird und in Koinzidenz mit dem später erscheinenden, periodisch auftretenden Signal oder einem entsprechenden Signalanteil miteinem charakteristischen Amplitudenverlaufs- oder Frequenzmuster ein Steuersignal abgegeben wird, wenn der Grad der Übereinstimmung der miteinander zu vergleichenden Muster einen vorgegebenen Wert überschreitet, wobei aus dem die Übereinstimmung anzeigenden Signal die Steuergröße hergeleitet wird, welche die Stimulationsgröße beeinflußt. Anstelle des Frequenzmusters kann dabei auch der zeitliche Verlauf eines oder mehrerer spektraler Anteile in dem Frequenzmuster, welches innerhalb eines Zeitsegments aufgenommen wurde, verglichen werden (Wavelet).

Die Steuergröße, welche die Zeitfolge oder den Zeitpunkt des Stimulationssignals beeinflußt, kann dabei insbesondere auch aus der Rate, der mittleren Häufigkeit, der zeitlichen Änderung der Rate oder dessen mittlerer Häufigkeit des eine Übereinstimmung anzeigenden Signals abgeleitet werden, so daß sich eine statistische Verdichtung der abgeleiteten zur Stimulation herangezogenen Information ergibt. Eine entsprechende Verdichtung der Information läßt sich auch erzielen, wenn die Information aus dem Muster des Amplitudenverlaufs und dem spektralen Muster gemeinsam ausgewertet werden.

Zur Erzeugung von Frequenzspektren wird das zeitabhängige Signal zweckmäßigerweise einer Fast-Fourier-Transformation (FFT) unterworfen.

Zusätzlich zur Beeinflussung des Steuersignals zur Steuerung der Zeitfolge des Stimulationssignals durch periodische Einflüsse kann es auch von Vorteil sein, das Steuersignal und damit das Stimulationssignal durch ein positives oder negatives Zufallssignal zu variieren. Die Stimulationsfrequenz des Schrittmachers wird also nicht völlig starr eingestellt, sondern variiert um den gewünschten Frequenzwert. Auch im gesunden menschlichen Körper ist die Herzfrequenz mehr oder weniger zufälligen Schwankungen unterworfen, die verschiedene Ursachen haben können. Solche Schwankungen sollten deshalb auch in einem Schrittmacher nachgebildet werden können. Wird dem Steuersignal ein verhältnismäßig kleiner Zufallswert überlagert, so kann hierdurch auch eine eventuelle positive Rückkoppelung im Regelkreis verhindert werden. Wird dem Steuersignal ein verhältnis großer Zufallswert überlagert, so kann hierdurch die hämodynamische Belastungsgrenze ertastet werden, wobei es dann notwendig ist, die Auswirkungen der Variationen auf eine im hämodynamischen Regelkreis befindliche Meßgröße zu erfassen. Durch die Variation der Stimulationsfrequenz können auch weitere Größen des hämodynamischen Regelkreises wie Zeitkonstanten und Proportionalität der Regelgrößen ermittelt werden, die wiederum zur Optimierung der Parameter des Ratenadaptionsalgorhythmus des Schrittmachers genutzt werden können.

Um einer möglichen Schwingneigung von Kreislauffunktionen innerhalb des Körpers des Patienten durch eine phasensynchrone Anregung infolge der erzeugten Herzstimulation entgegenzuwirken ist es zweckmäßig, wenn Mittel vorgesehen sind, welche bei Feststellung einer Amplitudenüberhöhung des aktuellen zeitlichen Mittelwertes des körpereigenen Rhythmussignals im Vergleich zu einem vorangehenden Langzeitmittelwert die Phasenlage der Änderung des Stimulationsgröße im Verhältnis zur Verlauf der zeitlichen Änderung der ermittelten Körperrrhythmusgröße um einen vorgegebenen oder einen statistisch ermittelten Phasenwinkel verändern.

Als körpereigene Sensorsignale, welche der erfindungsgemäßen Verarbeitung unterworfen werden, kommen insbesondere periodische Anteile des intrakardialen Elektrogramms, die intrakardiale oder transthorokale Impedanz, die Bluttemperatur, vagale/parasympathische oder barorezeptorische Nervensignale, der intraarterielle Druck oder dessen an zwei unterschiedlichen Meßorten aufgenommene Differenz als peripherer Gefäßwiderstand des zwischen den Meßorten befindlichen Teils des Kreislaufsystems oder das elektrochemische zelluläre lonenpotential in Betracht.

Andere vorteilhafte Weiterbildungen der Erfindung sind in den Unteransprüchen gekennzeichnet bzw. werden nachstehend zusammen mit der Beschreibung der bevorzugten Ausführung der Erfindung anhand der Figuren näher dargestellt.

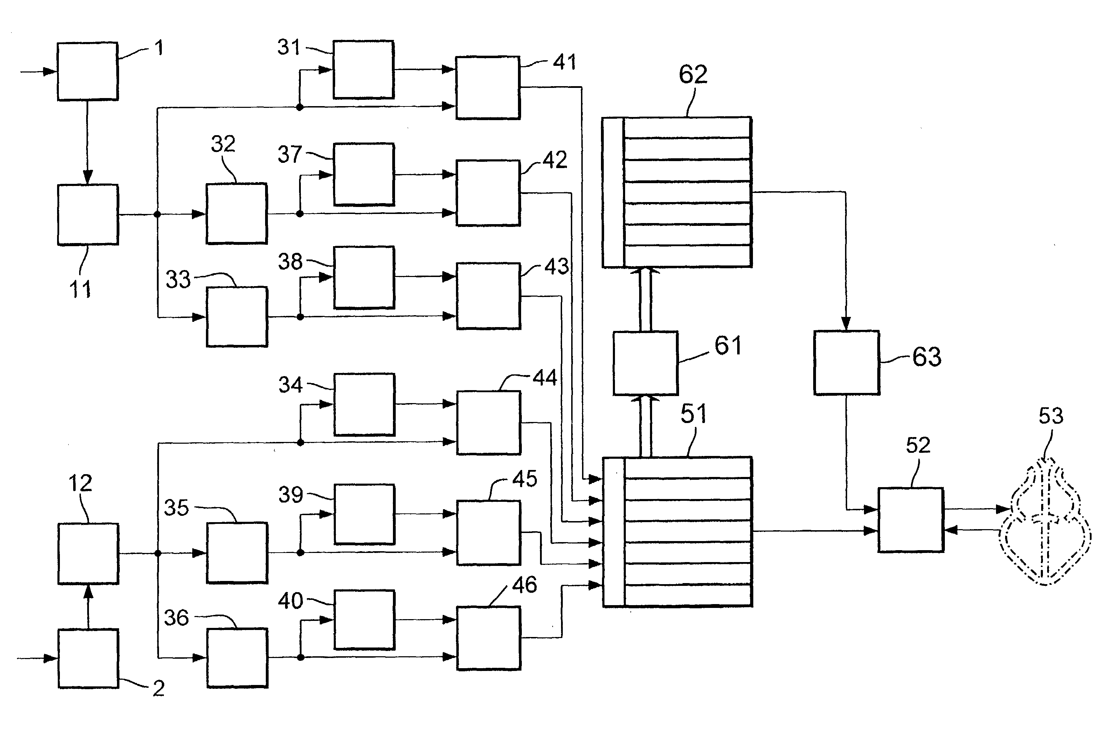

Die einzige Figur zeigt ein bevorzugtes Ausführungsbeispiel der Erfindung im Blockschaltbild.

Bei dem in der Figur dargestellten Ausführungsbeispiel handelt es sich um eine Vorrichtung zur Aufrechterhaltung oder Wiederherstellung eines natürlichen Herzrhythmus durch Erzeugung eines elektrischen Stimulationssignals, in Form eines implantierbaren Herzschrittmachers, welche mindestens einen innerhalb des Körpers des Patienten angeordneten Sensor zur Aufnahme eines eine Information über den Herzleistungsbedarf oder eine andere herzrelevante Größe enthaltenden körpereigenen Signals aufweist.

Dabei ist den Sensoren 1 und 2 eine aus mehreren nachfolgend zu beschreibenden Baugruppen bestehende Verarbeitungseinheit nachgeschaltet, welche aus den diesen Sensoren zugeführten (als Eingangspfeile dargestellten) Meßsignalen ein körpereigenes Rhythmussignal gewinnt, dessen Periodizität mindestens einige Sekunden umfaßt. Die Periode der Signale kann z. B. im Bereich der Atemfrequenz oder oberhalb liegen; sie liegt auf jeden Fall oberhalb der Periodizität der Herztätigkeit. Das körpereigene Rhythmussignal bildet mindestens mittelbar ein Steuersignal, das den Zeitpunkt oder die Zeitfolge und/oder den Zeitpunkt des Stimulationssignals beeinflußt.

Bei dem Sensor 1 soll es sich dabei um einen Blutdruckaufnehmer und bei dem Sensor 2 um eine Meßvorrichtung für den Thoraxwiderstand handeln, welche in üblichen Ausführung durch die beiden Elektroden einer monopolaren Herzschrittstimulationselektrode gebildet wird (ein Pol bildet die im Herzen verlegte Elektrode, während der Gegenpol durch das Stimulatorgehäuse im Brustraum gebildet wird).

Als Eingangssignale kommen aber auch das intrakardiale Elektrogramm, die intrakardiale oder transthorokale Impedanz, die Bluttemperatur, vagale/parasympathische oder barorezeptorische Nervensignale, der intraarterielle Druck oder dessen an zwei unterschiedlichen Meßorten aufgenommene Differenz, das elektrochemische zelluläre lonenpotential oder andere in Betracht.

Dazu erfolgt die Selektion des körpereigenen Rhythmussignals in den Baugruppen im oberen Teil der Figur in einem Frequenzbereich, der im wesentlichen dem Tag/Nacht-Rhythmus - und damit der inneren Uhr des Menschen - entspricht. Im unteren Teil der Figur erfolgt hingegen eine Selektion des körpereigenen Rhythmussignals im Bereich der Atemfrequenz oder einem Bruchteil der Atemfrequenz, d. h. in einem Frequenzbereich, der im wesentlichen einem 6 bis 20 Sekunden-Rhythmus, insbesondere 10 Sekunden-Rhythmus, entspricht.

In der Zeichnung sind in den Blöcken 1 1 und 12 Verstärkerschaltungen für die von den Sensoren aufgenommenen Signale vorgesehen. In Speicherbausteinen wird in Blöcken 31 und 34 der jeweils über ein Zeitsegment des Sensorsignals ermittelte Amplitudenverlauf gespeichert. In den Blöcken 41 und 44 erfolgt für die jeweiligen Signale innerhalb vorgegebener, an die Signalrhythmik angepaßter Zeitfenster eine Ermittlung der Übereinstimmung mit dem laufenden Signal, welches ebenfalls aus einem ausgewählten Amplitudenverlauf innerhalb eines Zeitsegments besteht. Bei festgestellter Übereinstimmung wird der Zeitabstand ermittelt und dieser als für die Rhythmik charakteristisches Signal weiterverarbeitet.

In den Stufen 32 und 35 wird eine Fast-Fourier-Anlalyse vorgenommen, so daß als Signalmuster für den Vergleich des aktuell erscheinenden mit einem in einem Verzögerungsglied 37 bzw. 39 zeitlich verzögerten Signal in den Stufen 42 bzw. 45 das mittlere Frequenzspektrum in dem jeweiligen Zeitsegment dient.

In den Stufen 33 und 36 werden statt dessen Wavelets aus dem laufenden Eingangssignal erzeugt und - nach Verzögerung in Verzögerungsgliedern 38, 40 - in den Stufen 43 und 46 ebenfalls miteinander verglichen, so daß aus der Wiederholung entsprechender Wavelets ebenfalls auf eine Periodizität des entsprechenden Sensorsignals geschlossen werden kann.

Hierbei wird in der Verarbeitungseinheit innerhalb eines mindestens mehrere Sekunden umfassenden Zeitsegments vorgegebener Dauer mindestens ein periodisch auftretendes Signal oder ein entsprechendr Signalanteil mit einem charakteristischen Amplitudenverlaufs- bzw. Frequenzmuster selektiert und festgehalten.

Das jeweils in dem Zeitsegment aufgenommene, periodisch auftretende Signal oder ein entsprechender Signalanteil mit einem charakteristischen Amplitudenverlaufsoder Frequenzmuster wird in den nachfolgenden Stufen gespeichert, das laufende Zeitsegment mit mindestens einem gleichartigen, zuvor aufgenommenen, periodisch auftretenden Signal oder ein entsprechender Signalanteil mit einem charakteristischen Amplitudenverlaufs- oder Frequenzmuster fortlaufend miteinander verglichen und in Koinzidenz mit dem später erscheinenden, periodisch auftretenden Signal oder einen entsprechenden Signalanteil mit einem charakteristischen Amplitudenverlaufs- oder Frequenzmuster ein Steuersignal abgegeben, wenn der Grad der Übereinstimmung der miteinander zu vergleichenden Muster einen vorgegebenen Wert überschreitet. Die Periodizität der rhythmischen Signale ergibt sich damit aus der Signalverzögerung bei aufeinanderfolgenden Signalen, welche notwendig ist, diese Signale (sei es der Amplitudenverlauf oder das Spektrum) mit ihrem jeweiligen Vorgänger nach einem Ähnlichkeits- oder Korrelationsverfahren "zur Deckung" zu bringen.

Die Ausgangssignale der Stufen 41 bis 46 stehen damit für die jeweils durch die Art der Verarbeitungsmittel 31 bis 46 bestimmte Periodizität der von den Sensoren 1 und 2 in den Eingangssignalen detektierten rhythmischen Signalanteile. Durch Adressierung eines nachfolgenden als Nachschlagetabelle ausgebildeten Speichers erfolgt die Kombination der für die Periodizität der Signale repräsentativen Ausgangssignale der Stufen 41 bis 46 als Adressierungssignale für den Speicher, der seinerseits die an den adressierten Speicherplätzen abgelegten Steuersequenzen an den konventionellen Herzstimulator oder Defibrillator abgibt. Dabei kann es sich um ein Ratensteuersignal oder um ein singuläres Signal zur Auslösung einer einzelnen Tachykardie-Terminierungs- oder Defibrillationssequenz handeln.

Es ist ersichtlich, daß durch Kombination der Ausgangssignale der Stufen 41 bis 46 auch komplexere Auswertungen der körperrhythmischen Signale vorgenommen werden können. Hierzu kommen insbesondere deren logische Verküpfungen, Überlagerungen, zeitliche Ableitungen, Häufungswerte etc. in Betracht.

Die Inhalte der adressierten Speicherplätze des Tabellenspeichers 51 geben ein Ausgangssignal an den - im übrigen konventionellen steuerbaren Stimulatorteil 52 ab, der in bekannter Weise in Wechselwirkung mit dem Herzen 53 steht. Durch die Steuersignale vom Ausgang des Speichers 51 wird im Falle eines ratengesteuerten Schrittmachers die Grundrate beeinflußt, während im Falle eines Defibrillators ein Defibrillationszyklus ausgeführt wird, wenn Körperrhythmiksignale einen vorgegebenen Schwellwert überschreiten.

Zusätzlich zu der Erzeugung eines körpereigenen Rhythmussignals zur Beeinflussung des Steuersignals, das den Zeitpunkt und/oder die Zeitfolge des Stimulationssignals beeinflußt, ist es auch zweckmäßig, dem Steuersignal einen zusätzlichen positiven oder negativen Zufallswert zu überlagern. Dies aus der Erkenntnis, daß auch im gesunden menschlichen Körper die Herzfrequenz mehr oder weniger zufälligen Schwankungen unterworfen ist. Diese Schwankungen können teilweise Ursachen in der Atemtätigkeit haben, so daß z. B. bei intensiver Atemtätigkeit in der Regel die Phasenlage der Herzkontraktionen durch die Atemtätigkeit bestimmt wird, nicht jedoch deren durchschnittliche Frequenz. Daneben ist auch die sogenannte respirative Arhythmie bekannt, die sich so äußert, daß die Herzfrequenz beim Einatmen kurzfristig steigt, beim Ausatmen dagegen sinkt. Andere Ursachen für solche Schwankungen sind beispielsweise im Zusammenwirken des sympathischen und parasympathischen Nervensystems oder im Zusammenwirken der Herzfrequenz und der Wasomotorik zu sehen.

Da der gesunde Körper zufällige Schwankungen der Herzfrequenz aufweist, ist zu vermuten, daß dieses Verhalten physiologisch bzw. hämodynamisch vorteilhaft ist, so daß aus diesem Grunde auch dieses Verhalten durch den Herzschrittmacher nachgebildet werden sollte.

Wird somit also die Stellgröße nichtstatisch geregelt, so ist in der Regel eine schnellere und flexiblere Antwort der Meßgröße zu erwarten, was wiederum zu einer schnelleren und flexibleren Anpassung der Stellgröße führen kann. Das Zusammenspiel zufällig auftretender Variationen kann zum beschleunigten Auffinden physiologisch günstiger Zustände des Regelkreises führen.

Bisher war davon ausgegangen, daß dem Steuersignal ein verhältnismäßig kleiner Zufallswert überlagert wird. Werden jedoch verhältnismäßig große Zufallswerte überlagert, so besteht die Möglichkeit, hämodynamische Belastungsgrenzen zu "ertasten". Dabei ist es notwendig, die Auswirkungen der Variationen auf eine im hämodynamischen Regelkreis befindliche Meßgröße zu erfassen, beispielsweise auf das Schlagvolumen, repräsentiert durch die intrakardiale Impedanz. Beispielsweise kann durch eine starke Erhöhung bzw. Verringerung der Stimulationsfrequenz das Schlagvolumen, mittels dessen das Kreislaufsystem die Variation der Herzfrequenz auszugleichen sucht, an seine untere bzw. obere Regelgrenze gebracht werden. Bei ichämiegefährdeten Patienten stellt die Stimulationsfrequenz bei Erreichen der Regelgrenze gleichzeitig ein Maß für die obere Grenzfrequenz dar, die zur Vermeidung von Ichemien nicht erreicht werden darf. Andere Diagnoseverfahren neben der Ichämiegrenzenerkennung sind ebenfalls auf der Basis der Regelgrenzen, die durch die Stimulationsfrequenzvariation erreicht werden, denkbar. Durch Variation der Stimulationsfrequenz können auch weitere Größen des hämodynamischen Regelkreises wie Zeitkonstanten und Proportionalität der Regelgrößen ermittelt werden, die wiederum zur Optimierung der Parameter des Ratenadaptionsalgorhythmus des Schrittmachers genutzt werden können.

Da es sich bei der Vorrichtung um eine solche handelt, bei der eine körpereigene Rhythmusgröße welche die Herzstimulation beeinflußt, durch die körperinterne Kopplung selbst wieder beeinflußt werden kann, kann - wie bei jedem Regelsystem - die Gefahr einer Schwingneigung gegeben sein. Zur Vermeidung sind deshalb Mittel vorgesehen, welche bei einer Überhöhung der mittleren Amplitude einer für die Kreislaufrhythmik charakteristischen Größe entsprechende Gegenmaßnahmen einleiten. Hierzu wird von den Stufen 41 bis 46 nicht nur der für die jeweilige innere Kreislaufrhythmik charakteristische Zeitabstand der aufeinanderfolgenden Mustersignale, sondern auch deren mittlere Amplitude zur Adressierung des Tabellenspeichers 51 benutzt. Überschreitet diese Amplitude einen vorgegeben Maximalwert, so wird über eine Schaltstufe 61 ein weiterer Tabellenspeicher 62 adressiert, welcher über eine Ausgangsstufe 63 eine Änderung der Stimulationsgröße im Verhältnis zum Verlauf der zeitlichen Änderung der ermittelten Körperrhythmusgröße um einen vorgegebenen oder einen statistisch ermittelten Phasenwinkel bewirkt.

Die Erfindung beschränkt sich in ihrer Ausführung nicht auf die vorstehend angegebenen bevorzugten Ausführungsbeispiele. Vielmehr ist eine Anzahl von Varianten möglich, welche von der dargestellten Lösung auch bei grundsätzlich anders gearteten Ausführungen Gebrauch machen.

## Patentansprüche

1. Vorrichtung zur Aufrechterhaltung oder Wiederherstellung eines natürlichen Herzrhythmus durch Erzeugung eines elektrischen Stimulationssignals, insbesondere in Form eines implantierbaren Herzschrittmachers, welche mindestens einen innerhalb des Körpers des Patienten anzuordnenden Sensor zur Aufnahme eines eine Information über den Herzleistungsbedarf oder eine andere herzrelevante Größe enthaltenden körpereigenen Signals aufweist, wobei dem Sensor eine Verarbeitungseinheit nachgeschaltet ist, welche ausgebildet ist, aus dem Meßsignal ein körpereigenes Rhythmussignal zu gewinnen, dessen Periodizität mindestens gleich der Periodizität der Atemtätigkeit ist und somit oberhalb der Periodizität der Herztätigkeit gelegen ist, wobei das körpereigene Rhythmussignal mindestens mittelbar ein Steuersignal bildet, das den Zeitpunkt und/oder die Zeitfolge des Stimulationssignals beeinflusst, wobei die Verarbeitungseinheit ausgebildet ist,
innerhalb eines mindestens mehrere Sekunden umfassenden Zeitsegments vorgegebener Dauer mindestens ein periodisch auftretendes Signal oder ein entsprechender Signalanteil mit einem charakteristischen Amplitudenverlaufs- oder Frequenzmuster zu selektieren und aus der Aufeinanderfolge des Auftretens dieses Musters das körpereigene Rhythmussignal herzuleiten wird, welches die Zeitfolge und/oder den Zeitpunkt des Stimulationssignals beeinflußt, wobei die aufeinanderfolgenden Zeitsegmente einen vorgegebenen Zeitabstand aufweisen, welcher der erwarteten Periodizität des Rhythmus entspricht,
das jeweils in dem Zeitsegment aufgenommene, periodisch auftretende Signal oder der entsprechende Signalanteil mit einem charakteristischen Amplitudenverlaufs- oder Frequenzmuster zu speichern,
das laufende Zeitsegment mit mindestens einem gleichartigen, zuvor aufgenommenen, periodisch auftretenden Signal oder ein entsprechender Signalanteil mit einem charakteristischen Amplitudenverlaufs- oder Frequenzmuster fortlaufend zu vergleichen **dadurch gekennzeichnet, daß** die Verarbeitungseinheit ferner ausgebildet ist,
bei Koinzidenz mit dem später erscheinenden, ein die Zeitdifferenz des Erscheinens anzeigendes Signal abzugeben, wenn der Grad der Übereinstimmung der miteinander zu vergleichenden Muster einen vorgegebenen Wert überschreitet, und aus dem die Zeitdifferenz anzeigenden Signal das körpereigene Rhythmussignal zu erzeugen.

2. Vorrichtung nach Anspruch 1, **dadurch gekennzeichnet, daß** die Selektion des körpereigenen Rhythmussignals in einem Frequenzbereich erfolgt, der im wesentlichen dem Tag/Nacht-Rhythmus entspricht.

3. Vorrichtung nach Anspruch 1, **dadurch gekennzeichnet, daß** die Selektion des körpereigenen Rhythmussignals in einem Frequenzbereich erfolgt, der im wesentlichen einem 6 bis 20 Sek, insbesondere 10 Sek-Rhythmus entspricht.

4. Vorrichtung nach Anspruch 1, **dadurch gekennzeichnet, daß** die Selektion des körpereigenen Rhythmussignals in einem Frequenzbereich erfolgt, der im wesentlichen der Atemfrequenz entspricht.

5. Vorrichtung nach Anspruch 1, **dadurch gekennzeichnet, daß** die Selektion des körpereigenen Rhythmussignals mittels Digitalfilterung oder der Anwendung einer Korrelationstechnik erfolgt.

6. Vorrichtung nach einem der vorhergehenden Ansprüche, **dadurch gekennzeichnet, daß** anstelle des Frequenzmusters der zeitliche Verlauf eines oder mehrerer spektraler Anteile in dem Frequenzmuster verglichen werden.

7. Vorrichtung nach Anspruch 6, **dadurch gekennzeichnet, daß** das körpereigene Rhythmussignal aus der zeitlichen Änderung des die Zeitdifferenz anzeigenden Signals abgeleitet wird.

8. Vorrichtung nach einem der vorhergehenden Ansprüche, **dadurch gekennzeichnet, daß** das körpereigene Rhythmussignal aus der Überlagerung oder Kumulation mehrerer aus der Rate, der mittleren Häufigkeit, der zeitlichen Änderung der Rate oder dessen mittlerer Häufigkeit des eine Übereinstimmung anzeigenden Signals für mindestens zwei Zeitsegmente unterschiedlicher Dauer erzeugt wird.

9. Vorrichtung nach einem der vorhergehenden Ansprüche, **dadurch gekennzeichnet, daß** die Steuergröße, welche die Zeitfolge oder den Zeitpunkt des Stimulationssignals beeinflußt, durch Überlagerung oder Kumulation mehrerer aus der Rate, der mittleren Häufigkeit, der zeitlichen Änderung der Rate oder dessen mittlerer Häufigkeit des eine Übereinstimmung anzeigenden Signals für mindestens zwei Zeitsegmente gleicher Dauer erzeugt wird, bei denen einerseits der Amplitudenverlauf und andererseits der Verlauf des Frequenzmusters ausgewertet wird.

10. Vorrichtung nach einem der vorhergehenden Ansprüche, **dadurch gekennzeichnet, daß** das Frequenzmuster einer Fast-Fourier-Transformation (FFT) unterworfen wird.

11. Vorrichtung nach einem der vorhergehenden Ansprüche, **dadurch gekennzeichnet, daß** es sich bei dem Steuersignal, welches die Zeitfolge oder den Zeitpunkt des Stimulationssignals beeinflußt, um die Stimulationsrate oder ein Tachykardieterminierungs- bzw. Defibrillationssignal handelt.

12. Vorrichtung nach einem der vorhergehenden Ansprüche, **dadurch gekennzeichnet, daß** dem Steuersignal, das die Zeitfolge oder den Zeitpunkt des Stimulationssignals beeinflußt, ein zusätzlicher positiver oder negativer Zufallswert überlagert wird.

13. Vorrichtung nach Anspruch 12, **dadurch gekennzeichnet, daß** ein verhältnismäßig kleiner Zufallswert überlagert wird.

14. Vorrichtung nach Anspruch 12, **dadurch gekennzeichnet, daß** ein verhältnismäßig großer Zufallswert überlagert wird.

15. Vorrichtung nach einem der vorhergehenden Ansprüche, **dadurch gekennzeichnet, daß** Mittel vorgesehen sind, welche bei Feststellung einer Amplitudenüberhöhung des aktuellen zeitlichen Mittelwertes des körpereigenen Rhythmussignals im Vergleich zu einem vorangehenden Langzeitmittelwert die Phasenlage der Änderung des Stimulationsgröße im Verhältnis zum Verlauf der zeitlichen Änderung der ermittelten Körperrhythmusgröße um einen vorgegebenen oder einen statistisch ermittelten Phasenwinkel verändern.

16. Vorrichtung nach einem der vorhergehenden Ansprüche, **dadurch gekennzeichnet, daß** Vergleiche von Mustern, Spektren oder Signalverläufen in der Verarbeitungseinheit nach der Methode der kleinsten Fehlerquadrate ausgeführt werden.

17. Vorrichtung nach einem der vorhergehenden Ansprüche, **dadurch gekennzeichnet, daß** es sich bei dem körpereigenen Signal um das intrakardiale Elektrogramm, die intrakardiale oder transthorokale Impedanz, die Bluttemperatur, vagale/parasympathische oder barorezeptorische Nervensignale, den intraarteriellen Druck oder dessen an zwei unterschiedlichen Meßorten aufgenommene Differenz, das elektrochemische zelluläre lonenpotential handelt.

## Claims

1. Device for maintaining or re-establishing a natural heart rhythm by generating an electrical stimulation signal, in particular a device in the form of an implantable heart pacemaker that comprises at least one sensor arranged inside the patient's body for picking up a body-specific signal containing information concerning demand for heart performance, or another magnitude relevant to the heart, wherein connected downstream of the sensor is a processing unit that obtains from the measurement signal a body-specific rhythm signal whose periodicity is at least equal to the periodicity of the breathing activity, and thus lies above the periodicity of the heart activity, wherein the body-specific rhythm signal, at least indirectly, forms a control signal that influences the point in time and/or the time interval of the stimulation signal, wherein the processing unit is constructed: to select within a time segment of a predetermined duration and comprising at least several seconds at least one periodically-appearing signal or a corresponding signal portion having a characteristic amplitude response or frequency pattern, and to derive the body-specific rhythm signal from the succession of the appearance of this pattern, which signal influences the time interval and/or the point in time of the stimulation signal, the sequential time segments exhibiting a predetermined interval that corresponds to the expected perodicity of the rhythm, to store the periodically-occurring signal picked up in each case in the time segment, or the corresponding signal portion having a characteristic amplitude response or frequency pattern, to continually compare the current time segment with at least one similar, previously picked-up, periodically-occurring signal, or a corresponding signal portion having a characteristic amplitude response or frequency pattern, **characterised in that** the processing unit is also constructed, in coincidence with the later-appearing signal, to emit a signal that indicates the time difference of appearance when the degree of conformity of the patterns to be compared with one another exceeds a predetermined value, and to generate from the signal indicating the time difference the body-specific rhythm signal.

2. Device according to claim 1, **characterised in that** the body-specific rhythm signal is selected in a frequency range that substantially corresponds to the day/night rhythm.

3. Device according to claim 1, **characterised in that** the body-specific rhythm signal is selected in a frequency range that substantially corresponds to a 6 to 20 s rhythm, in particular a 10 s rhythm.

4. Device according to claim 1, **characterised in that** the body-specific rhythm signal is selected in a frequency range that substantially corresponds to the breathing frequency.

5. Device according to claim 1, **characterised in that** the body-specific rhythm signal is selected by means of digital filtering or the application of a correlation technique.

6. Device according to any one of the preceding claims, **characterised in that** instead of the frequency pattern, the temporal course of one or several spectral portions in the frequency pattern are compared.

7. Device according to claim 6, **characterised in that** the body-specific rhythm signal is derived from the temporal change of the signal indicating the time difference.

8. Device according to any one of the preceding claims, **characterised in that** the body-specific rhythm signal is generated from the superimposition or accumulation of a plurality from among the rate, the average frequency, the temporal change of the rate or its average frequency, of the signal indicating conformity, for at least two time segments of different duration.

9. Device according to any one of the preceding claims, **characterised in that** the control magnitude that influences the time interval or point in time of the stimulation signal is generated by the superimposition or accumulation of several from among the rate, the average frequency, the temporal change of the rate or its average frequency, of the signal indicating conformity, for at least two time segments of different duration, during which is evaluated the amplitude response, on the one hand, and the course of the frequency pattern, on the other hand.

10. Device according to any one of the preceding claims, **characterised in that** the frequency pattern is subjected to a Fast-Fourier-Transformation (FFT).

11. Device according to any one of the preceding claims, **characterised in that** the control signal that influences the time interval or the point in time of the stimulation signal is the stimulation rate of a tachycardia-terminating or defibrillation signal.

12. Device according to any one of the preceding claims, **characterised in that** an additional positive or negative random value is superimposed on the control signal, which influences the time interval or the point in time of the stimulation signal.

13. Device according to claim 12, **characterised in that** a relatively small random value is superimposed.

14. Device according to claim 12, **characterised in that** a relatively large random value is superimposed.

15. Device according to any one of the preceding claims, **characterised in that** means are provided that, on determination of an excessive rise in the amplitude of the current temporal mean value of the body-specific rhythm signal in comparison to a preceding long-term mean value, change the phase position of the change of the stimulation magnitude, in relation to the course of the temporal change of the determined body rhythm magnitude, by a predetermined or statistically-determined phase angle.

16. Device according to any one of the preceding claims, **characterised in that** comparisons of patterns, spectra or signal characteristics are made in the processing unit using the method of the smallest error square.

17. Device according to any one of the preceding claims, **characterised in that** the body-specific signal is the intracardial electrogram, the intracardial or transthoracic impedance, blood temperature, vagal/parasympathetic or baroreceptive nerve signals, intra-arterial pressure or the pressure difference picked up at two different measurement locations, or the electrochemical cellular ionic potential.

## Revendications

1. Procédé pour maintenir ou rétablir un rythme cardiaque naturel par production d'un signal de stimulation électrique, notamment sous la forme d'un stimulateur cardiaque implantable, qui comporte au moins un capteur devant être disposé à l'intérieur du corps du patient pour enregistrer un signal propre au corps, qui comprend une information concernant le besoin de puissance cardiaque ou une autre grandeur important pour le coeur, et dans lequel en aval du capteur est branchée une unité de traitement agencée de manière à obtenir, à partir du signal de mesure, un signal de rythme propre au corps et dont la périodicité est au moins égale à la périodicité de l'activité respiratoire et par conséquent est réglée de manière à être supérieure à la périodicité de l'activité cardiaque, et dans lequel le signal de rythme propre au corps forme au moins indirectement un signal de commande, qui influe sur l'instant et/ou la succession dans le temps du signal de stimulation, et dans lequel l'unité de traitement est agencée de manière à sélectionner, en l'espace d'un segment temporel d'une durée prédéterminée, comprenant au moins plusieurs secondes, au moins un signal apparaissant périodiquement et une partie de signal correspondante possédant un modèle caractéristique d'allure en amplitude ou en fréquence, et de dériver, de la succession de l'apparition de ce modèle, le signal de rythme propre au corps et qui inclut sur la séquence temporelle et/ou l'instant du signal de stimulation, les segments temporels successifs étant séparés par un intervalle de temps prédéterminé, qui correspond à la périodicité attendue du rythme,
mémoriser le signal qui apparaît périodiquement et est reçu dans le segment temporel, ou la partie correspondante du signal présentant un modèle caractéristique d'allure en amplitude ou en fréquence,
comparer de façon permanente le segment temporel courant à au moins un signal identique apparaissant périodiquement, reçu auparavant, ou une partie correspondante de signal présentant un modèle caractéristique d'allure en amplitude ou en fréquence,
**caractérisé en ce que** l'unité de traitement est en outre agencée
de manière à délivrer, en cas de coïncidence avec le signal apparaissant ultérieurement, un signal indiquant la différence entre les temps d'apparition, lorsque le degré de concordance des modèles à comparer entre eux dépasse une valeur prédéterminée, et produire à partir du signal indiquant la différence de temps, le signal de rythme propre au corps.

2. Dispositif selon la revendication 1, **caractérisé en ce que** la sélection du signal de rythme propre au corps s'effectue dans une gamme de fréquences, qui correspond essentiellement au rythme jour/nuit.

3. Dispositif selon la revendication 1, **caractérisé en ce que** la sélection du signal de rythme propre au corps s'effectue dans une gamme de fréquences, qui correspond essentiellement au rythme de 6 à 20 secondes, notamment 10 secondes.

4. Dispositif selon la revendication 1, **caractérisé en ce que** la sélection du signal de rythme propre au corps s'effectue dans une gamme de fréquences qui correspond essentiellement à la fréquence respiratoire.

5. Dispositif selon la revendication 1, **caractérisé en ce que** la sélection du signal de rythme propre au corps s'effectue au moyen d'un filtrage numérique ou de l'utilisation d'une technique de corrélation.

6. Dispositif selon l'une des revendications précédentes, **caractérisé en ce qu'**à la place du modèle de fréquences, on compare la variation dans le temps de l'une de la pluralité de composantes spectrales dans le modèle de fréquences.

7. Dispositif selon la revendication 6, **caractérisé en ce que** le signal de rythme propre au corps est dérivé de la variation dans le temps du signal indiquant la différence de temps.

8. Dispositif selon l'une des revendications précédentes, **caractérisé en ce que** le signal de rythme propre au corps est produit à partir de la transmission ou du cumul de plusieurs éléments tirés de la cadence, de la fréquence moyenne, de la variation dans le temps de la cadence ou de sa fréquence moyenne du signal, indiquant une concordance, pour au moins deux segments de temps ayant des durées différentes.

9. Dispositif selon l'une des revendications précédentes, **caractérisé en ce que** la grandeur de commande, qui influe, par superposition ou cumul de plusieurs éléments tirés de la cadence, de la fréquence moyenne, de la variation dans le temps de la cadence ou de la fréquence moyenne du signal indiquant une concordance, pour au moins deux segments de temps de même durée, dans lesquels d'une part l'allure de l'amplitude et d'autre part l'allure du modèle de fréquence sont évalués.

10. Dispositif selon l'une des revendications précédentes, **caractérisé en ce que** le modèle de fréquence est soumis à une transformation de Fourier rapide (FFT).

11. Dispositif selon l'une des revendications précédentes, **caractérisé en ce que** dans le cas du signal de commande, qui influe sur la séquence temporelle ou l'instant du signal de stimulation, il s'agit de la cadence de stimulation ou d'un signal de détermination d'achèvement de tachycardie ou de défibrillation.

12. Dispositif selon l'une des revendications précédentes, **caractérisé en ce qu'**au signal de commande, qui influe sur la séquence temporelle ou l'instant du signal de stimulation, est superposée une valeur aléatoire supplémentaire positive ou négative.

13. Dispositif selon la revendication 12, **caractérisé en ce que** la superposition est réalisée avec une valeur comparativement faible.

14. Dispositif selon la revendication 12, **caractérisé en ce qu'**on utilise pour la superposition une valeur aléatoire comparativement élevée.

15. Dispositif selon l'une des revendications précédentes, **caractérisé en ce qu'**il est prévu des moyens, qui, lors de la détermination d'un accroissement de l'amplitude de la valeur moyenne actuelle dans le temps du signal de rythme propre au corps par rapport à une valeur moyenne précédente de longue durée, modifient la position de phase de la modification de la grandeur de stimulation par rapport à l'allure de la variation dans le temps de la grandeur déterminée du rythme du corps, et ce d'un angle de phase prédéterminé ou d'un angle de phase déterminé statistiquement.

16. Dispositif selon l'une des revendications précédentes, **caractérisé en ce que** des comparaisons de modèles, de spectres ou de variations de signaux sont exécutées dans l'unité de traitement selon la méthode des moindres carrés des erreurs.

17. Dispositif selon l'une des revendications précédentes, **caractérisé en ce qu'**en ce qui concerne le signal propre au corps, il s'agit de l'électrogramme intracardiaque, de l'impédance intracardiaque ou de la température du sang, de signaux nerveux de type vagals / parasympathiques ou baroréceptifs, de la pression intraartérielle ou de sa différence enregistrée en deux emplacements différents de mesure, du potentiel ionique électrochimique cellulaire.
